# EUROPEAN PATENT APPLICATION

(11) **EP 4 375 671 A1**
(43) Date of publication of application: **29.05.2024**
(21) Application number: 22210427.5
(22) Date of filing: 30.11.2022
(51) Int. Cl.: G01N 35/00, G01N 35/04, G01N 21/64, G01N 33/543, G01N 33/53

(54) **DOUBLE DIAGNOSTIC DEVICES AND DOUBLE DIAGNOSTIC METHODS**

(30) Priority: 22.11.2022 KR 20220157412
(71) Applicant: InTelos Inc., Daegu 41566 (KR); M1 International Inc., Daejeon 34214 (KR)
(72) Inventor: LEE, Joo Hyun, 35216 Daejeon (KR); LEE, Sang Heon, 05698 Seoul (KR); KIM, Seon Tae, 41561 Daegu (KR)
(74) Representative: Milli, Simone

(57) **Abstract**

This Invention is aimed to provide a dual diagnostic device capable of quickly confirming the diagnosis result from a cartridge, improving accuracy through double-placed sensors, and increasing test efficiency by continuously performing a series of test, and may include:
a movement module with rails formed thereon, a sensor module fixed on the upper part of the movement module, a recognition module that acquires information from a cartridge transported by the movement module, a control section that controls the operation of the movement module and the sensor module, and a result analysis section to analyze information obtained from the sensor modules.

## Description

### [Technology field]

The present invention is related to a diagnostic device, and more specifically, to providing a dual diagnostic device which allows two measurements, one using a fluorescent sensor and one an optical sensor, in order to improve the recognition rate of an agent reaction section displaying the diagnostic result.

### [Technology behind the invention]

The general problem of devices used for diagnosing infection diseases is that users have difficulty confirming the diagnosis result. The result is that users may be left unable to clearly understand the diagnostic result.

If the disease concerned is highly contagious and users do not clearly recognize his or her own infection, it will boost the spread of infection within the community.

To address this problem, Patent Registration No. 10-1891049 is intended to provide an immunodiagnostic cartridge capable of improving diagnostic accuracy. However, an electric pad must be installed in the cartridge, a technology that requires additional modifications to a standard cartridge.

Furthermore, when a highly contagious disease breaks out, testing a large number of people urgently is necessary occasionally, which requires a technique for quickly processing multiple tests.

### [Content of the Invention]

### [Targets of Solution]

A contrivance derived from aforesaid technical context, this Invention is intended to provide a dual diagnostic device which can quickly check the diagnosis result of the cartridge, improve the accuracy through the double-formulated sensor, and increase the inspection efficiency by continuously carrying out a number of tests consecutively.

### [Means of Solution]

WHEREAS, in order to achieve the aforesaid purpose, this Invention may include a movement module for reciprocating a cartridge, a sensor module fixed to an upper portion of aforesaid movement module to detect a measurement result of aforesaid cartridge, a recognition module that obtains information from the cartridge, a control section for controlling the operation of the aforesaid movement module and a result analyzing section that analyzes information obtained from aforesaid sensor module.

WHEREAS, the aforesaid sensor module further includes an optical sensor, a fluorescence sensor, and an illuminator embedded into aforesaid sensor module, and can improve the recognition rate of aforesaid optical sensor and fluorescent sensor as the reagent reaction section which displays the test result of aforesaid cartridge accommodates the light radiated from aforesaid illuminator.

WHEREAS, the aforesaid light includes an LED light and a UV lamp, the former casting blue light to increase the recognition rate of the optical sensor, and the latter flickering to improve the recognition rate of the fluorescent sensor.

WHEREAS, the aforesaid sensor module may be recognized by the aforesaid optical sensor and florescent sensor in sequence depending on the type of the cartridge.

WHEREAS, the aforesaid sensor module may include an optical sensor that recognizes light and a fluorescent sensor recognizing a fluorescent substance, have a sensor port created into which aforesaid optical sensor and fluorescent sensor are inserted, and include a module body equipped with an illuminator port into which the illuminator casting light to the cartridge is inserted.

WHEREAS, the aforesaid module body may be so formed that a plural number of the sensor holes are formed such that the optical sensor and the fluorescent sensor are vertically inserted into the module body, and a plural number of lighting holes are inserted into aforesaid module body at a preset angle.

WHEREAS, the module body may include in its lower section a contact surface that contacts the aforesaid cartridge, and may block the light illuminating the reagent reaction section on which the test result from the cartridge is displayed.

WHEREAS, the aforesaid movement module may include a prop that moves along the rail, as well as a rail motor that moves the prop according to a command from the controller.

WHEREAS, the prop may include a tray coupled to the aforesaid cartridge, the tray may include a lower plate of tray connecting the tray to the upper portion of the aforesaid prop, and a cartridge holding part may be formed to which the cartridge is connected in the lower part of the tray.

WHEREAS, the aforesaid recognition module may include a proximity sensor for ensuring that the aforesaid cartridge is inserted into the aforesaid inspecting section, and a barcode module for recognizing a 2D barcode denoting the type of the cartridge, as well as a temperature sensor for checking the current temperature condition.

WHEREAS, the control section may adjust the distance by which the aforesaid movement module moves the aforesaid cartridge, by the barcode module identifying the type of the cartridge.

WHEREAS, in regard to the dual diagnostic device which many include: a cartridge which can display the test result; a movement module into which the cartridge is inserted; a proximity sensor which ensures that the cartridge is inserted into the aforesaid movement module; a barcode module which obtains information from the cartridge; a rail motor which moves aforesaid cartridge by mobilizing aforesaid movement module; a reagent reaction section displaying the test result from the aforesaid cartridge; a temperature sensor measuring the temperature of the aforesaid reagent reaction part; a light casting light to the aforesaid reagent reaction part; an optical sensor and a fluorescent sensor obtaining the aforesaid test result; a system UI in which the aforesaid user enters a command; and a control section for controlling the action of aforesaid rail motor; the stage where the user selects a single measurement mode and a continuous measurement mode through the aforesaid system UI, according to the number of cartridges put into the dual diagnostic machine may be included.

WHEREAS, the aforesaid single measurement mode may include: the stage where the aforesaid cartridge is inserted into the aforesaid movement module; the stage where the aforesaid cartridge is recognized by the aforesaid proximity sensor; the stage where the 2D barcode formed in the aforesaid cartridge is recognized by the aforesaid barcode module; the stage where the aforesaid control section chooses between an optical cartridge measurement mode and a fluorescent cartridge measurement mode, depending on the type of the aforesaid cartridge recognized by the aforesaid barcode module; the stage where the aforesaid temperature sensor measures the temperature of the aforesaid reagent reaction part; and the stage where the aforesaid optical sensor measures the contamination of the aforesaid reagent reaction part.

WHEREAS, the aforesaid optical cartridge measurement mode may include: the stage where the aforesaid rail motor moves the aforesaid cartridge to the lower part of the aforesaid optical sensor; the stage where the image of the aforesaid reagent reaction section is measured by the aforesaid optical sensor; the stage where the aforesaid controlling part, once measurement of aforesaid optical sensor is completed, moves aforesaid cartridge to the lower part of aforesaid fluorescent sensor; the stage where the aforesaid fluorescent sensor measures the amount of fluorescence in the aforesaid reagent reaction part; and the stage where the user removes the aforesaid cartridge.

WHEREAS, the aforesaid fluorescent cartridge measurement mode may include: the stage where the aforesaid rail motor moves the aforesaid cartridge to the lower part of the aforesaid fluorescent sensor; the stage where the aforesaid fluorescent sensor measures the amount of fluorescence in the aforesaid reagent reaction part; the stage where the aforesaid controlling part, once measurement of aforesaid fluorescent sensor is completed, moves aforesaid cartridge to the lower part of aforesaid optical sensor; and the stage where the user removes the aforesaid cartridge.

WHEREAS, the aforesaid continuous measurement mode may include: the stage where the aforesaid cartridge is inserted into the aforesaid movement module; the stage where the 2D barcode formed in the aforesaid cartridge is recognized by the aforesaid barcode module; the stage where the aforesaid control section chooses between an optical cartridge measurement mode and a fluorescent cartridge measurement mode, depending on the type of the aforesaid cartridge recognized by the aforesaid barcode module; the stage where the aforesaid temperature sensor measures the temperature of the aforesaid reagent reaction part; and the stage where the aforesaid optical sensor measures the contamination of the aforesaid reagent reaction part.

WHEREAS, the optical cartridge measurement mode may include: the stage where the aforesaid rail motor moves the aforesaid cartridge to the lower part of the aforesaid optical sensor; the stage where the image of the aforesaid reagent reaction section is measured by the aforesaid optical sensor; the stage where the aforesaid controlling part, once measurement of aforesaid optical sensor is completed, moves aforesaid cartridge to the lower part of aforesaid fluorescent sensor; the stage where the aforesaid fluorescent sensor measures the amount of fluorescence in the aforesaid reagent reaction part; the stage where the user removes the aforesaid cartridge; the stage where the aforesaid proximity sensor recognizes that the already removed aforesaid cartridge is inserted back again; and the stage where the aforesaid control section stops the action of the dual diagnostic device when the user enters a stop command through the system UI.

WHEREAS, the aforesaid fluorescent cartridge measurement mode may include: the stage where the aforesaid rail motor moves the aforesaid cartridge to the lower part of the aforesaid fluorescent sensor; the stage where the aforesaid fluorescent sensor measures the amount of fluorescence in the aforesaid reagent reaction part; the stage where the stage where the aforesaid controlling part, once measurement of aforesaid fluorescent sensor is completed, moves aforesaid cartridge to the lower part of aforesaid optical sensor; the stage where the image of the aforesaid reagent reaction section is measured by the aforesaid optical sensor; the stage where the user removes the aforesaid cartridge; the stage where the aforesaid proximity sensor recognizes that the already removed aforesaid cartridge is inserted back again; and the stage where the aforesaid control section stops the action of the dual diagnostic device when the user enters a stop command through the system UI.

### [Effects of the Invention]

According to an embodiment of this Invention, the dual diagnostic device can improve the rate of recognition on the user's status of infection by analyzing the recognized information through the sensor module.

According to an embodiment of this Invention, the dual diagnostic device may use illuminator to improve the recognition rate of the fluorescent sensor and the optical sensor.

According to an embodiment of this Invention, the dual diagnostic device can use two types of lighting, LED lighting and UV lamp.

According to an embodiment of this Invention, the dual diagnostic device may be recognized by an optical sensor and a fluorescent sensor sequentially depending on the type of cartridge.

According to an embodiment of this Invention, the dual diagnostic device may be installed with the optical module and the fluorescent sensor adjoining the sensor module.

According to an embodiment of this Invention, the dual diagnostic device may have the optical sensor and the fluorescent sensor installed vertically, and the module body installed at a slant in the illuminator hole.

According to an embodiment of this Invention, the dual diagnostic device has a contact side created, which can block the light to the reagent reaction section of the cartridge.

According to an embodiment of this Invention, a movement module in which a prop moves along the rail may be created in the dual diagnostic device.

According to an embodiment of this Invention, the dual diagnostic device may have a tray which, in combination with the prop, transports the cartridge.

According to an embodiment of this Invention, the dual diagnostic device may include a recognition module that recognizes the 2D barcode created in the cartridge.

According to an embodiment of this Invention, the controller may adjust the movement quantity of the cartridge in the dual diagnostic device.

According to an embodiment of this Invention, the dual diagnostic method enables the user to choose a single measurement mode or a continuous measurement mode.

According to an embodiment of this Invention, as for the optical cartridge, the dual diagnostic method enables measurement by the fluorescent sensor, after measurement was made with respect to the optical sensor.

According to an embodiment of this Invention, as for the fluorescent cartridge, the dual diagnostic method enables measurement by the optical sensor, after measurement was made with respect to the fluorescent sensor.

According to an embodiment of this Invention, the dual diagnostic method makes it possible that the user may, by the continuous measurement mode, measure the cartridge continuously until it is stopped through the system UI.

### [Brief Description of Drawings]

Figure 1 is an internal side view of a dual diagnostic device according to an embodiment of this Invention;
Figure 2 is a perspective view of a mobile module according to an embodiment of this Invention;
Figure 3 is a plan-view of a tray according to an embodiment of this Invention;
Figure 4 is a side view of a sensor module according to an embodiment of this Invention;
Figure 5 is a view showing the movement of the diagnostic kit in the dual diagnostic device according to an embodiment of this Invention;
Figure 6 is a perspective view of the housing for diagnostic device according to an embodiment of this Invention;
Figure 7 is a partial cut-away view of a cartridge according to an embodiment of this Invention;
Figure 8 is an operation flowchart of a single measurement mode according to an embodiment of this Invention.
Figure 9 is an operation flowchart of a continuous measurement mode according to an embodiment of this Invention.

### [Specific Details for Implementing this Invention]

In this section, I will explain in detail the desirable embodiments of this Invention by reference to the Figures provided below.

Advantages and features of this Invention, and a method of achieving them will become clear by referring to the following examples, together with the accompanying Figures. However, this Invention is not limited to the embodiments disclosed below, but will be implemented in diversified forms. The embodiments presented here are intended to complete the disclosure of this Invention and to fully inform those with ordinary knowledge in the technology field of the scope of this Invention. This Invention is only defined by the classifications of the claims.

In describing this Invention, a detailed explanation on any related publicly known technique will be omitted to the extent that I determine might obscure the summary of this Invention.

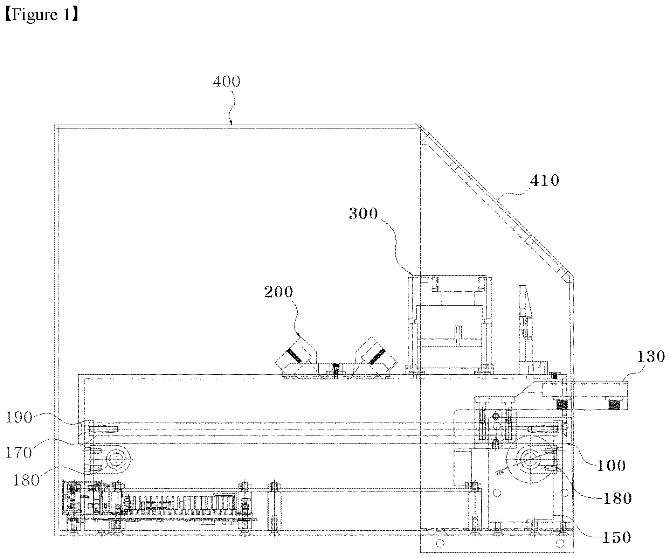

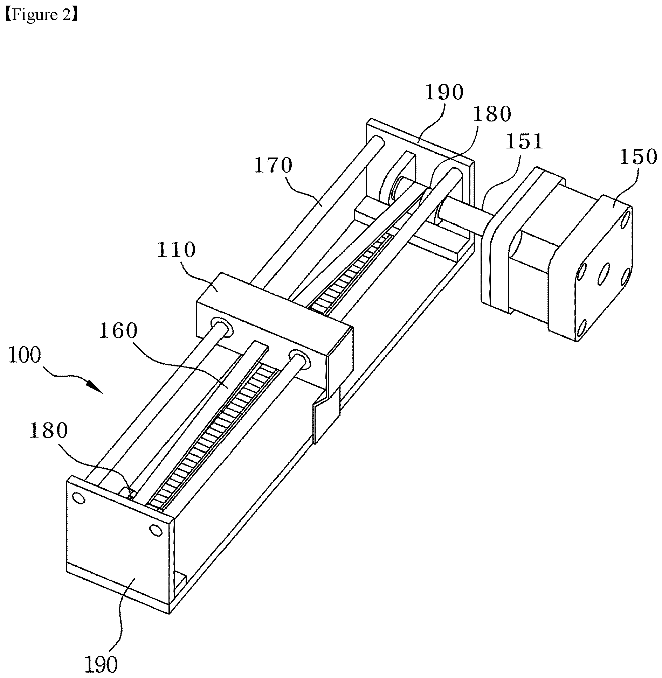

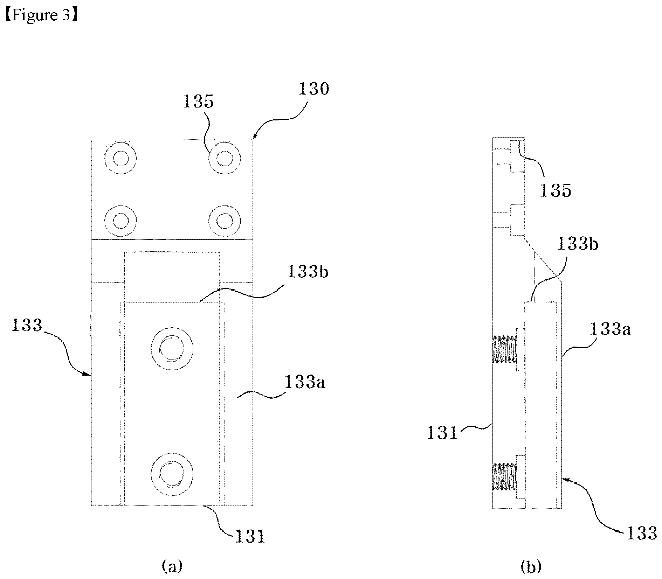

Figure 1 shows a side view of a dual diagnostic device, Figure 2 shows a perspective view of a mobile module, and Figure 3 shows a plan-view of the tray, according to an embodiment of this Invention, respectively.

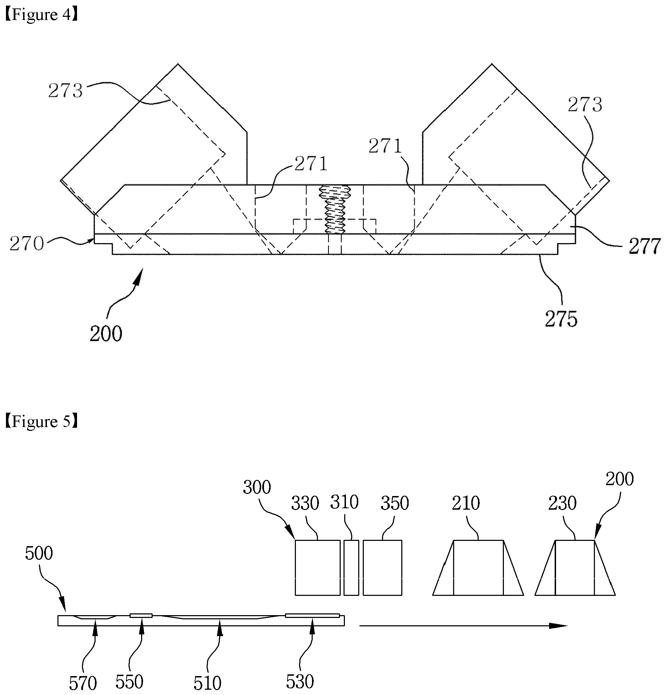

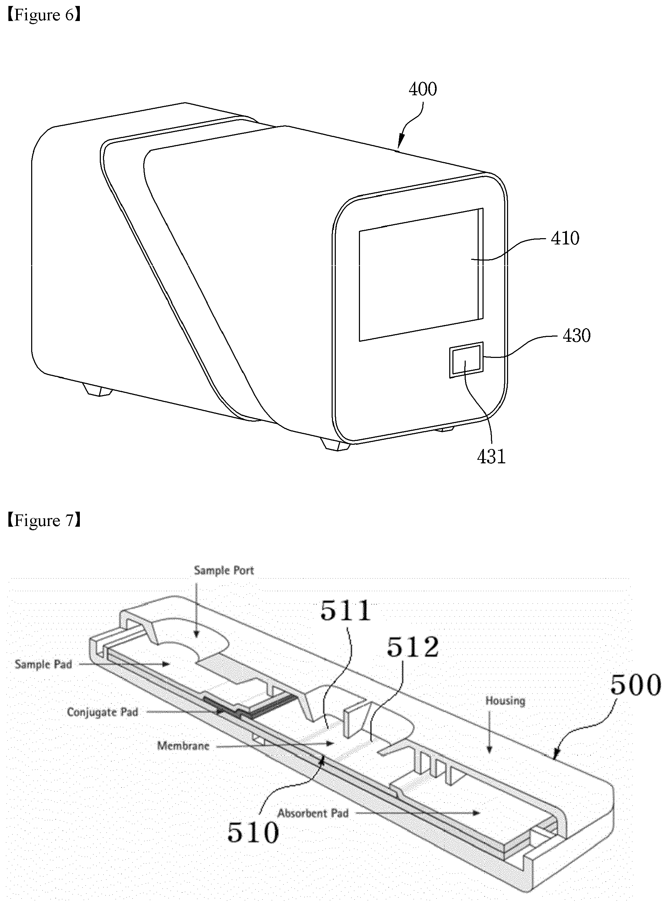

Figure 4 shows a side view of the sensor module, Figure 5 shows a diagram depicting the movement of the diagnostic kit in the dual diagnostic device, Figure 6 shows a perspective view of the housing of diagnostic device, and Figure 7 shows a cut-away view of a cartridge, according to an embodiment of this Invention, respectively.

First, I will describe the composition of the cartridge used in the embodiment of this Invention, with reference to Figure 7.

As shown in Figure 7, the cartridge of this Invention may include a fluorescent substance. The fluorescent substance may be formed of materials such as quantum dot or europium.

By means of this, it is possible to solve the problem that the recognition rate decreases when the user checks whether he or she is positive or negative through the control line (511) and the experiment line (512) displayed in the reagent reaction section (510) which displays the test result.

If the user supplies the sample to the reagent drop section (570) of the cartridge (500), the experiment line (512) may appear if the test result is positive. In this case, depending on the reactivity of the sample used, the test result may not appear clearly.

At this time, the fluorescent substance may generate fluorescence at a position where the inspection line is generated by the fluorescent substance, and the inspection result can be clearly recognized since even a very small amount of the fluorescent substance can be easily identified.

In addition, in order to more clearly identify the fluorescence generated from the fluorescent substance in the experiment line (512), a UV lamp may be used, and the inspection result is more readily readable to the user.

The dual diagnostic device (1) by this Invention may be largely composed of a mobile module (100), a sensor module (200), a recognition module (300), and a housing of diagnostic device (400).

As shown in Figure 1, the dual diagnostic device (1) by this Invention is so configured that a cartridge (500) is transported following the aforesaid movement module (100), and the aforesaid cartridge (500) is recognized by the sensor module (200) and the recognition module (300).

As shown in Figure 1 and 2, the dual diagnostic device (1) of this Invention may have the movement module (100) created, on which the rail (170) is formed. The aforesaid movement module (100) plays the role to transfer the cartridge (500) in the direction of the recognition module (300) and the sensor module (200).

The aforesaid movement module (100) may be provided with a prop (110) to enable transportation in the longitudinal direction. The aforesaid prop (110) is combined with the aforesaid cartridge (500) so that the prop (110) of the movement module (100) can move along the rail.

As shown in Figure 2, the movement module (100) of this Invention may be composed of a rail (170), rail holder (190), prop (110), power transmission belt (160), and rail motor (150). By means of this, the cartridge (500) can be moved according to the command from the control section (600).

The aforesaid rail (170) is formed in the shape of a rod, and is connected through the aforesaid prop (110) so that the aforesaid prop (110) can be transported along the aforesaid rail (170). The aforesaid rail (170) may be fixed by connecting both ends between a pair of rail holders (190).

The aforesaid prop (110) may be reciprocated back and forth along the aforesaid rail (170) by a rail motor (150). With its rotation shaft (151) and the transfer wheel (180) connected to the power transmission belt (160), the aforesaid rail motor (150) can relay the rotatory power.

As shown in Figure 2, both ends of the power transmission belt (160) are adjoining the prop (110), with its middle section hooked on the aforesaid rotating shaft and the aforesaid transfer wheel (180), so that the aforesaid prop (110) moves along the aforesaid rail (170) by the rotation of the aforesaid rotating shaft.

In the upper portion of the aforesaid prop (110), a fixing port (not shown) is formed, so that it can be connected to the tray (130) into which the user inserts the cartridge (500).

As shown in Figure 6, the aforesaid tray (130) partially protrudes outside the inlet formed in the housing of diagnostic device (400), more specifically, toward the cartridge inlet (430), and therefore the user can easily insert the cartridge (500).

The aforesaid tray (130) may have a lower plate of tray (131) having a screw port (135) connected to the prop (110).

On the aforesaid lower plate of tray (131), there may be formed a cartridge holding part (133) into which the cartridge (500) is inserted. The aforesaid cartridge holding part (133) may be formed as a space between the fixing guides (133a) protruding from both ends of the lower plate of tray (131).

The lower plate of tray (131) may contain an insertion terminal (133b) formed in an ending shape, and the cartridge holding part (133) may be formed from one end of the lower plate of tray (131) to the insertion terminal (133b).

Through this arrangement, the insertion terminal (133b) can stop the slide movement of the inserted cartridge (500), ensuring that the cartridge (500) is always fixed at the correct position, moving the correct distance according to the command of the control section (600).

In addition, the height of the fixing guide (133a) may be higher than the height of the cartridge (500), through which the side of the lower part of module body (277) of the sensor module (200) contacts the fixing guide (133a), aiding the contact surface (275) to make the reagent reaction section (510) a blackout shade.

At this point, I will describe the configuration of the aforesaid sensor module (200) by reference to the Figures.

As shown in Figure 1, the aforesaid sensor module (200) is provided on the upper side of the aforesaid transfer module (200) and plays the role to read the cartridge (500) located on the prop (110).

The aforesaid sensor module (200) may be provided with a sensor port (271) into which the sensors (210, 230) are inserted. In this embodiment, the optical sensor (210) and the fluorescent sensor (230) may be inserted into the sensor module (200).

The optical sensor (210) and the fluorescent sensor (230) are so configured as to recognize the test result appearing in the reagent reaction section (510) of the cartridge (500).

The sensor module (200) may include an illuminator port (273), which may be formed in a slant at a preset angle. Through this, it is possible to lower the height of the sensor module (200) even when a relatively large illuminator (250) is inserted, and the aforesaid illuminator (250) can cast light to the side of the reagent reaction section (510).

The optical sensor (210) and the fluorescent sensor (230) may acquire the result of the reagent reaction section (510) at a position perpendicular to the reagent reaction section (510), thereby obtaining an improved recognition rate.

A plate-shaped contact area (275) may be formed on the lower portion of the sensor module (200), and the contact area (275) may be in contact with the cartridge (500) to form the reagent reaction section (510) as a blackout shade. That is, the cartridge (500) is enveloped by the fixing guide (133a) of the aforesaid tray (131) and the contact area (275), forming a blackout shade. As a result, the light from an external light source other than the light cast from the illuminator (250) is effectively blocked, thereby improving the recognition rate.

As shown in Figure 1, multiple illuminators (250) may be used, and may be composed of an LED light and a UV lamp in this embodiment.

The aforesaid LED light can be used together with the optical sensor (210), and the aforesaid UV lamp can be used together with the fluorescent sensor (230).

The LED light can radiate blue light to the reagent reaction section (510) of the aforesaid cartridge (500), because when blue light is cast to the optical sensor (210), it can be recognized more clearly than with white light.

The aforesaid UV lamp is operated in the mode of blinking, generating the continuous reaction of the fluorescent substance in the aforesaid reagent reaction section (510), so that the fluorescent sensor (230) can more accurately examine the fluorescent substance.

The dual diagnostic device (1) may obtain a test result by using an optical sensor and a fluorescent sensor sequentially depending on the type of the cartridge (500).

That is, if the cartridge (500) is of a type which detects the fluorescent substance better, the test result is first obtained through the fluorescent sensor (230), while using the information obtained through the optical sensor (210) as ancillary information. As a corollary, if the cartridge (500) is of a type which can better obtain information through optical sensor (210), the test result may be first obtained through the optical sensor (210), while using the information obtained from the fluorescent sensor (230) as ancillary information.
the sensor module (200) may be fixed to the housing of diagnostic device (400), and the movement module (100) may be created below the sensor module (200).

As a result, the sensor module (200) can be located at the same position at all times, and thus the control section (600), which controls the operation of the movement module (100), makes a move in a preset distance, so that the reagent reaction section (510) can be correctly positioned below the optical sensor (210) and the fluorescent sensor (230).

As shown in Figure 4, the sensor module (200) of this Invention may form a module body (270) having a rectangular contact area (275) formed thereon, and a lower part of module body (277) including the contact area (275).) Shaped in trapezoid, the lower part of module body (277) has a thicker central portion, and both ends of it may be formed in a slanted shape.

A pair of the illuminator holes (273) may be formed at both ends of the slanted ends, and the sensor port (271) may be created in a thick central portion.

The illuminator port (273) is formed at a preset angle, so that the illuminator (250) inserted into the illuminator port (273) can cast light to the center part of the sensor module (200).

The pair of illuminator holes (273) is designed to accept an LED light into one of its sides and a UV lamp into the other side. The sensor port (271) is formed in a pair, one may accept the optical sensor (210), while the other may accept the fluorescent sensor (230).

Through this arrangement, when the optical sensor (210) obtains the test result of the reagent reaction section (510), the LED light can cast light, and when the fluorescent sensor (230) obtains the test result from the reagent reaction section (510), the UV lamp may start flickering.

Although not shown in a Figure, fixed plates are formed at both ends of the module body (270), which can be connected to the housing of diagnostic device (400).

When the sensor module (200) is inserted as a result of the downward movement of the cartridge (500), and the sensor module (200) confirms the test result of the reagent reaction section (510), the cartridge (500) may be detached again.

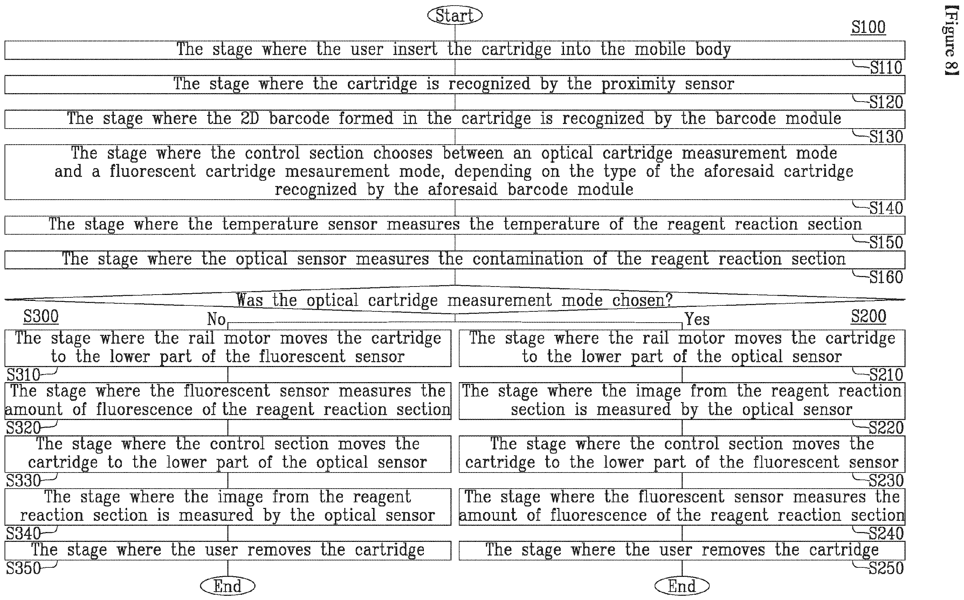

Figure 8 is an operation diagram of a dual diagnostic device according to an embodiment of this Invention.

As shown in Figure 8, a 2D barcode (530) is created on one end of the upper part of the cartridge (500), followed by creation of a reagent reaction section (510) and a handwriting section (550) in a sequence, and a reagent drop section (570) can be formed at the other end.

As a result, when the cartridge (500) is inserted, the recognition module (300) can firstly obtain information from the cartridge (500) by 2D barcode (530).

In relation to the recognition module (300), a proximity sensor (310) that confirms insertion of the cartridge (500) is created, followed by creation of a barcode module (330) that obtains information of the 2D barcode (530), as well as creation of the temperature sensor (350) that measures the temperature of the reagent reaction section (510).

The 2D barcode (530) may record information such as the type and the effective period of the cartridge (500), through which it is possible to obtain necessary information before checking the test result from the cartridge (500).

When the cartridge (500) is inserted into the dual diagnostic device (1), with its insertion direction reversed, the proximity sensor (310) may confirm the insertion of the cartridge (500), but the barcode module (330) may fail to obtain the information from the 2D barcode (530).

In this case, the control section (600) may determine that the cartridge (500) is inserted in the reverse direction, and activate the movement module (100) to move the cartridge (500) inward by the length of the cartridge (500), so that 2D barcode (530) can be read by the barcode module (330).

The control section (600) may reset the quantum of the cartridge (500) movement to be in line with the reversed direction.

The handwriting section (550) can be used for the user to enter additional information, and equipped with an additional photo sensor (370) to store the information of the handwritten section (550), which can be printed out together with the test result.

Before the sensor module (200) acquires information on the reagent reaction section (510), the photo sensor (370) may check whether the reagent reaction section (510) is contaminated, which makes it possible to avoid having a deviant result.

The cartridge (500) may be moved to the lower part of the recognition module (300) and the sensor module (200) in a sequel. After obtaining the test result from the cartridge (500) via the sensor module (200), the cartridge (500) is moved toward the recognition module (300) again, so that the user can remove the cartridge (500).

Figure 6 is a perspective view of the housing of the diagnostic device according to an embodiment of this Invention.

As shown in Figure 6, on the front surface of the housing of diagnostic device (400), a display (410) may be created, which may output a system UI (411). Through the system UI (411), the user can enter a command to the dual diagnostic device (1), and can obtain the diagnosis result as well as information from the dual diagnostic device (1).

A cartridge inlet (430) may be created on the front surface of the housing of diagnostic device (400). Through the cartridge inlet (430), the user may insert the cartridge (500) into the inside of the dual diagnostic device (1).

The tray (130) may, by the movement module (100), protrude to the outside of the housing of diagnostic device (400) through the cartridge inlet (430). By this design, the user can easily insert or remove the cartridge (500) into or from the tray (130).

An inlet shielding plate (431) is formed at the cartridge inlet (430) such that when the tray (130) protrudes, it pushes the inlet shielding plate (431) keeping itself protruded to the outside of the housing of diagnostic device (400), and when movement module (100) sends the tray back into the inside, the inlet shielding plate (431) may return to its original position.

In this case, one side of the inlet shielding plate (431) is combined with the housing of diagnostic device (400) in a way capable of rotation, and can maintain the state of closure by operation of an elastic medium such as a spring, and when the tray (130) pushes the inlet shielding plate (431), the inlet shielding plate (431) starts rotation for opening.

When the tray (130) moves back into the housing of diagnostic device (400), the inlet shielding plate (431) rotates again by the elastic medium to block the cartridge inlet 430. This makes it possible to block light from entering the housing of diagnostic device (400).

A power line can be connected to the rear of the housing of diagnostic device (400), a power button to enable power on and off function of the dual diagnostic device (1) is formed, and it can be connected to an information transmission cable that transmits information measured by the dual diagnostic device.

Figure 8 is an operation flowchart of a single measurement mode according to an embodiment of this Invention.

As shown in Figure 8, the single measurement mode of the optical cartridge and the fluorescent cartridge (S100) may include a stage where the cartridge is inserted by the user (S110) and the stage where the proximity sensor (310) confirms the insertion of the cartridge (500) (S120). In this way, it is possible to check whether the measurement process is being performed normally.

When the proximity sensor has detected the insertion of the cartridge (500), the barcode module (330) may perform a stage (S130) of recognizing the 2D barcode (530) formed in the cartridge (500). As a result, it is possible to check the type of the 2D barcode (530) and the effective period of the cartridge.

Upon completion of the stage of recognizing the 2D barcode (530) (S130), the control section (600) can proceed with the optical cartridge measurement mode (S200) if the inserted cartridge (500) is an optical cartridge, and proceed with the fluorescent cartridge measurement mode (S300) if it is a fluorescent cartridge.

Upon completion of the stage to choose between the optical cartridge measurement mode (S200) and the fluorescent cartridge measurement mode (S300) (S140), the cartridge (500) is moved by the movement module (100), followed by a stage where the temperature sensor (350) measures the temperature of the reagent reaction section (S150).

A stage may be performed where the photo sensor (370) measures the contamination of the reagent reaction section (510) (S160). Through this process, it is possible to determine whether there is an irregularity in the cartridge (500) before the sensor module (200) measures the reagent reaction section (510).

If it is determined that there is no irregularity in the reagent reaction section (510) and the inserted cartridge (500) is an optical cartridge, the optical cartridge measurement mode (S200) may be processed, and the stage may proceed where the rail motor (150) moves the cartridge (500) to the lower part of the optical sensor (210) (S210).

When the cartridge (500) is moved to the lower part of the optical sensor (210), the stage may proceed where the LED illuminator (250) radiates blue light to the reagent reaction section (510), and the optical sensor (210) measures the image of the reagent reaction section (510) (S220). In this case, the blue light may improve the recognition rate of the optical sensor (210).

When the stage of measuring the image by the optical sensor (210) (S220) is completed, the stage of moving the cartridge (500) to the lower part of the fluorescent sensor (230) (S230) will start, followed by the stage where the UV lamp flickers, and the fluorescent sensor measures the amount of fluorescence (S240). In this case, the UV lamp may show the highest recognition rate when it is in the mode of flickering.

Upon completion of the stage where the fluorescent sensor (230) measures the amount of fluorescence (S240), the cartridge (500) may be discharged to the outside of the housing of diagnostic device (400) by the movement module (100), and the user can remove the cartridge (500).

When the fluorescent cartridge measurement mode (S300) is implemented, a stage may be initiated (S310) where the cartridge (500) moves to the lower part of the fluorescent sensor (230) by the movement module (100), followed by the stage where the fluorescent sensor (230) measures the amount of fluorescence in the reagent reaction section (510) (S320).

Upon completion of measurement of the fluorescent sensor (230), the stage where the control section (600) may move the cartridge (500) to the lower part of the optical sensor (S330) may proceed, followed by a stage where the optical sensor (210) measures the image of the reagent reaction section (510) (S340).

In this case, when the fluorescent sensor (230) measures the amount of fluorescence, the UV lamp may start flickering, and when the optical sensor (210) measures the image, the LED illuminator (250) may cast blue light to the reagent reaction section (510).

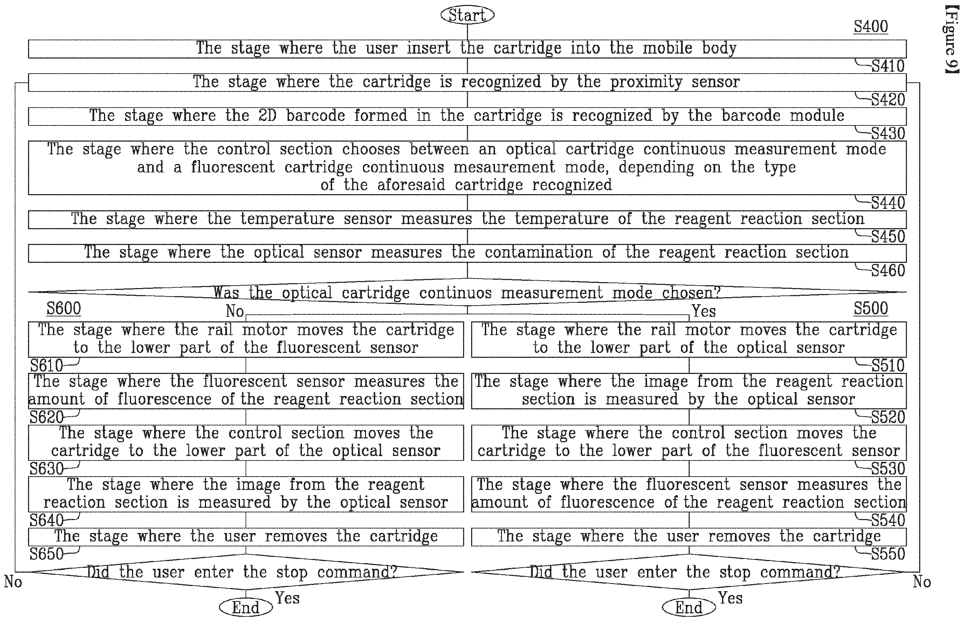

Figure 9 is an operation flowchart of a continuous measurement mode according to an embodiment of this Invention.

As shown in Figure 9, when the user gives a command to the dual diagnostic device (1) to implement the continuous measurement mode (S400) through the system UI 411, the stage where cartridge (500) is inserted into the movement module (100) (S410) proceeds, followed by the stage where the proximity sensor (310) confirms the insertion of the cartridge (500) (S420).

The stage where the 2D barcode (530) formed in the cartridge (500) recognizes the barcode module (330) (S430) may proceed. As a result, it is possible to obtain information such as the type and effective period of the cartridge (500).

The stage may proceed where a selection is made between the optical cartridge continuous measurement mode (S500) and the fluorescent cartridge continuous measurement mode (S600) according to the obtained information of the cartridge (500) (S440). As a result, a test process appropriate for the type of cartridge (500) may be carried out, thereby improving the recognition rate.

The stage may proceed where the temperature sensor (350) measuring the temperature of the reagent reaction section (510) (S450), followed by the stage of measuring the contamination of the reagent reaction section through the photo sensor (370) (S460). In this way, it is possible to confirm whether an irregularity has occurred in the test result of the reagent reaction section (510).

When the optical cartridge continuous measurement mode (S500) proceeds, the stage where the rail motor (150) moves the cartridge (500) to the lower part of the optical sensor (210) (S510) commences, followed by the stage where the optical sensor (210) measures the image of the reagent reaction section (510) (S520).

By casting blue light to the reagent reaction section (510), the LED lighting may improve the recognition rate of the optical sensor (210).

Upon completion of the stage where the optical sensor (210) measures the image (S520), the stage may start where the control section (600) moves the cartridge (500) to the lower part of the fluorescent sensor (230) (S530), and the fluorescent sensor (230) may measure the amount of fluorescence in the reagent reaction section (510) (S540).

The UV lamp, while flickering, repeats the reaction of the fluorescent substance so that the fluorescent sensor (230) can more accurately measure the amount of fluorescence of the reagent reaction section (510).

As a result, it is possible to draw a more accurate diagnosis result by adding the fluorescence amount information to the image information measured by the optical sensor (210).

Upon completion of the test of the cartridge (500), the movement module (100) may discharge the first cartridge (500) inserted into the dual diagnostic device (1) to the outside. The user may perform the stage of removing the discharged cartridge (500) (S470).

A stage may proceed where the proximity sensor (310) recognizes that the cartridge (500) is removed and reinserted again by the user (S420).

These stages may be continuously repeated as many times as necessary for the user, and when the user enters a stop command through the system UI (411), the stage may start where the control section (600) stops the operation of the dual diagnostic device (1) (S480).

The control section (600) chooses between the optical cartridge continuous measurement mode (S500) and the fluorescent cartridge continuous measurement mode (S600) according to the type of the cartridge (500) recognized by the barcode module (330) (S440). In that stage, the fluorescent cartridge measurement mode (S600) may proceed if the cartridge (500) is determined to be a fluorescent cartridge.

When the fluorescent cartridge continuous measurement mode (S600) proceeds, the stage may proceed where the movement module (100) moves the cartridge (500) to the lower part of the fluorescent sensor (S610).

When the cartridge (500) is positioned on the lower part of the fluorescent sensor, the UV lamp starts flickering, followed by a stage where the fluorescent sensor (230) measures the amount of fluorescence of the reagent reaction section (510) (S620).

Upon completion of measuring the amount of fluorescence, the cartridge (500) may be moved to the lower part of the optical sensor (210) by the movement module (100) (S630), and the LED illuminator (250) casts blue light to the reagent reaction section, followed by the stage where the optical sensor (210) measures the image of the reagent reaction section (510) (S640).

In this process, the recognition rate of the inspection result can be improved by adding image information measured by the optical sensor to the amount of fluorescence measured by the fluorescent sensor (230).

Upon completion of the measurement of the image by the optical sensor, the movement module (100) may discharge the cartridge (500) to the outside, followed by the stage where the user may remove the discharged cartridge (500) from the tray (300) (S470).

A stage proceeds where the proximity sensor (310) recognizes that the user has reinserted the cartridge (500) (S420), and this stage may be repeated as many times as the user needs.

A stage proceeds where the control section (600) stops the operation of the dual diagnostic device (1) as the user enters a stop command through the system UI (S480), which marks completion of all the measurement stages.

This Invention has been described with reference to the embodiment(s) shown in the Figures, and as such the description is only exemplary. One who has ordinary knowledge of technology in the relevant field would understand that a diversity of modifications may be made to the embodiment examples and the whole or part of the embodiments illustrated above can be selectively combined for new configuration. Hence, the true gamut of technical protection of this Invention must be defined by the technological ideas underlying the claims appended hereto.

## Claims

1. A dual diagnostic device with the following characteristics:
a movement module for reciprocating the cartridge
a sensor module fixed to the upper part of the movement module to draw the measurement result of the cartridge;
a recognition module for obtaining information on the cartridge;
a control section for controlling the operation of the movement module and the sensor module; and the result analysis section for analyzing the information obtained from the sensor modules.

2. A dual diagnostic device with the following characteristics:
In relation to Claim 1,
The aforesaid cartridge includes fluorescent substance such as europium or quantum dot, and aforesaid fluorescent substance which generates fluorescent light at a position where the experiment line of the reagent reaction section is formed displaying the test result, thereby increasing the recognition rate.

3. A dual diagnostic device with the following characteristics:
In relation to Claim 1,
The aforesaid sensor module further includes an optical sensor, a fluorescent sensor, and an illuminator inserted into the sensor module, and
the light from the aforesaid illuminator is cast to the reagent reaction section displaying the test result of the aforesaid cartridge, thereby increasing the recognition rate of the optical sensor and the fluorescent sensor.

4. A dual diagnostic device with the following characteristics:
In relation to Claim 3,
The aforesaid illuminator includes an LED light and a UV lamp, the former casting blue lights to increase the recognition rate of the aforesaid optical sensor, and the latter flickering to improve the recognition rate of the aforesaid fluorescent sensor.

5. A dual diagnostic device with the following characteristics:
In relation to Claim 3,
The aforesaid sensor module changes the order in which the aforesaid cartridge is recognized by the optical sensor and the fluorescent sensor depending on the type of the cartridge.

6. A dual diagnostic device with the following characteristics:
In relation to Claim 1,
The aforesaid sensor module includes an optical sensor that recognizes light and a fluorescent sensor that recognizes fluorescent substance, and
includes a module body having multiple sensor holes into which the optical sensor and the fluorescent sensor are inserted at a close distance, and an illuminator port accepting an illuminator casting light to the cartridge.

7. A dual diagnostic device with the following characteristics:
In relation to Claim 6,
The module body is so configured that Multiple sensor holes are formed to allow the optical sensor and the fluorescent sensor to be inserted in a direction perpendicular to the ground, multiple illuminator holes are formed to allow the aforesaid illuminator to be inserted at a preset angle to the ground.

8. A dual diagnostic device with the following characteristics:
In relation to Claim 6,
The aforesaid module body is so configured to include a contact area adjoining the aforesaid cartridge at a lower part and **characterized by** the process whereby the aforesaid contact area and aforesaid cartridge are so closely adjacent that the light is blocked to the reagent reaction section which displays the aforesaid test result from the aforesaid cartridge.

9. A dual diagnostic device with the following characteristics:
In relation to Claim 1,
The aforesaid mobile module includes a prop moving along the rail, and
a rail motor that moves the prop according to a command from the control section.

10. A dual diagnostic device with the following characteristics:
In relation to Claim 9,
The prop
includes the tray connected to the aforesaid cartridge, and
the aforesaid tray includes a lower plate of tray connected to the upper part of the aforesaid prop, and aforesaid lower plate of tray forms a cartridge holding part on which the cartridge is fixed.

11. A dual diagnostic device with the following characteristics:
In relation to Claim 1,
The aforesaid recognition module
includes a proximity sensor that confirms that the aforesaid cartridge is inserted into the aforesaid dual diagnostic device, includes a barcode module that recognizes a 2D barcode that records the type of aforesaid cartridge, and includes a temperature sensor that confirms the temperature of the reagent reaction section that displays the result from the aforesaid cartridge.

12. A dual diagnostic device with the following characteristics:
In relation to Claim 11,
The aforesaid control section
controls the distance in which the aforesaid movement module moves the aforesaid cartridge depending on the type of the cartridge, which has been confirmed by the aforesaid barcode module.

13. A dual diagnostic method with the following characteristics:
In relation to a dual diagnostic device which includes:
a cartridge which displays the test result;
a movement module into which the cartridge is inserted;
a proximity sensor which confirms that the cartridge is inserted into the movement module;
a barcode module that obtains information from the cartridge;
a rail motor that activates the movement module to move the cartridge;
a reagent reaction section that displays the test result from the cartridge;
a temperature sensor which measures the temperature of the reagent reaction section;
an illuminator which casts light to the reagent reaction section;
an optical sensor and a fluorescence sensor that obtain the test result;
the system UI where the user enters commands; and
a control section that controls the operation of the rail motor;
and includes a stage where the user chooses between a single measurement mode and a continuous measurement mode through the system UI, depending on the number of cartridges put in the dual diagnostic device.

14. A dual diagnostic method with the following characteristics:
In relation to Claim 13,
The aforesaid single measurement mode includes:
the stage where the user inserts the aforesaid cartridge into the aforesaid movement module;
the stage where aforesaid cartridge is recognized by the proximity sensor;
the stage where the 2D barcode formed in the cartridge is recognized by the barcode module;
the stage where the aforesaid control section chooses between the optical cartridge measurement mode and the fluorescent cartridge measurement mode depending on the type of the cartridge recognized by the barcode module;
the stage where the aforesaid temperature sensor measures the temperature of the reagent reaction section; and
the stage where the aforesaid optical sensor measures contamination of the reagent reaction section.

15. A dual diagnostic method with the following characteristics:
In relation to Claim 4,
The aforesaid optical cartridge measurement mode includes:
the stage where the aforesaid rail motor moves the aforesaid cartridge to the lower part of the optical sensor;
the stage where an image from the aforesaid reagent reaction section is measured by the aforesaid optical sensor;
the stage where, upon completion of the measurement by the aforesaid optical sensor, the aforesaid control section moves the aforesaid cartridge to a lower part of the aforesaid fluorescent sensor;
the stage where the aforesaid fluorescence sensor measures the amount of fluorescence in the aforesaid reagent reaction section; and
the stage where the user removes the aforesaid cartridge.

16. A dual diagnostic method with the following characteristics:
In relation to Claim 14,
The aforesaid fluorescent cartridge measurement mode includes:
the stage where the aforesaid rail motor moves the cartridge to a lower part of the aforesaid fluorescent sensor;
the stage where the aforesaid fluorescence sensor measures the amount of fluorescence of the aforesaid reagent reaction section;
the stage where, upon completion of measurement by the aforesaid fluorescent sensor, the aforesaid control section moves the aforesaid cartridge to a lower part of the aforesaid optical sensor;
the stage where the aforesaid optical sensor measures the image from the aforesaid reagent reaction section; and
the stage where the user removes the aforesaid cartridge.

17. A dual diagnostic method with the following characteristics:
In relation to Claim 13,
The aforesaid continuous measurement mode includes:
the stage where the user inserts the aforesaid cartridge into the aforesaid movement module;
the stage where the aforesaid cartridge is recognized by the proximity sensor;
the stage where the 2D barcode formed on the aforesaid cartridge is recognized by the aforesaid barcode module;
the stage where the aforesaid control section chooses between the optical cartridge continuous measurement mode and the fluorescent cartridge continuous measurement mode, depending on the type of cartridge recognized by the aforesaid barcode module;
the stage where the aforesaid temperature sensor measures the temperature of the aforesaid reagent reaction section; and
the stage where and contamination of the aforesaid reagent reaction section is measured by the aforesaid optical sensor.

18. A dual diagnostic method with the following characteristics:
In relation to Claim 17,
The aforesaid optical cartridge continuous measurement mode includes:
the stage where the aforesaid rail motor moves the cartridge to the lower part of the optical sensor;
the stage where the image from the aforesaid reagent reaction section is measured by the aforesaid optical sensor;
the stage where, upon completion of the measurement by the aforesaid optical sensor, the aforesaid control section moves the aforesaid cartridge to a lower part of the aforesaid fluorescent sensor;
the stage where the aforesaid fluorescence sensor measures the amount of fluorescence in the aforesaid reagent reaction section;
the stage where the user removes the aforesaid cartridge;
the stage where the aforesaid proximity sensor recognizes that the aforesaid cartridge, once removed, is inserted again; and
the stage where the aforesaid control section stops the operation of the aforesaid dual diagnostic device when the user enters a stop command through the system UI.

19. A dual diagnostic method with the following characteristics:
In relation to Claim 17,
The fluorescent cartridge continuous measurement mode includes:
the stage where the aforesaid rail motor moves the cartridge to the lower part of the fluorescent sensor;
the stage where the image from the aforesaid reagent reaction section is measured by the aforesaid fluorescent sensor;
the stage where, upon completion of the measurement by the aforesaid fluorescent sensor, the aforesaid control section moves the aforesaid cartridge to a lower part of the aforesaid optical sensor;
the stage where the aforesaid optical sensor measures the image from the aforesaid reagent reaction section;
the stage where the user removes the aforesaid cartridge;
the stage where the aforesaid proximity sensor recognizes that the aforesaid cartridge, once removed, is inserted again; and
the stage where the aforesaid control section stops the operation of the aforesaid dual diagnostic device when the user enters a stop command through the system UI.
